(19) Europäisches Patentamt / European Patent Office / Office européen des brevets

(11) **EP 3 552 545 B1**

(12) **FASCICULE DE BREVET EUROPEEN**

(45) Date de publication et mention de la délivrance du brevet:
**03.11.2021 Bulletin 2021/44**

(51) Int Cl.:
*A61B 5/055* *(2006.01)*   *A61B 5/30* *(2021.01)*
*G01R 33/389* *(2006.01)*   *A61B 5/00* *(2006.01)*
*G01R 33/567* *(2006.01)*   *G01R 33/565* *(2006.01)*

(21) Numéro de dépôt: **19168194.9**

(22) Date de dépôt: **09.04.2019**

(54) **PROCÉDÉ ET DISPOSITIF DE CORRECTION EN TEMPS RÉEL DE CHAMP MAGNÉTIQUE**

VERFAHREN UND VORRICHTUNG ZUR MAGNETFELDKORREKTUR IN ECHTZEIT

METHOD AND DEVICE FOR REAL-TIME MAGNETIC FIELD CORRECTION

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **12.04.2018 FR 1853201**

(43) Date de publication de la demande:
**16.10.2019 Bulletin 2019/42**

(73) Titulaires:
• **Schiller Medical**
**67160 Wissembourg (FR)**
• **Université de Lorraine**
**54052 Nancy Cedex (FR)**
• **Centre Hospitalier Régional Universitaire de Nancy**
**54035 Nancy (FR)**

(72) Inventeurs:
• **FELBLINGER, Jacques**
**54850 Méréville (FR)**
• **GUILLOU, André**
**67160 Wissembourg (FR)**
• **PETITMANGIN, Grégory**
**67480 Leutenheim (FR)**

(74) Mandataire: **Cabinet Bleger-Rhein-Poupon**
**4a rue de l'Industrie**
**67450 Mundolsheim (FR)**

(56) Documents cités:
WO-A1-2007/073576   WO-A1-2012/107692
WO-A2-2008/155488   US-A1- 2009 270 715
US-A1- 2018 055 417

## Description

La présente invention se situe dans le domaine de la mesure de données physiologiques de patients par acquisition de signaux électro-physiologiques en temps réel. Plus particulièrement, le recueil des signaux se fait dans un environnement particulier susceptible de générer des artéfacts sur le signal. Plus spécifiquement encore, le contexte peut-être celui de l'acquisition de signaux enregistrant l'activité d'organes tels que le cœur, le cerveau etc. dans un environnement fortement exposé à des champs magnétiques statiques et commutés comme par exemple produit par un dispositif d'imagerie médicale de type IRM (imagerie à résonance magnétique) auquel est également soumis le patient.

En pratique, étant donné que le principe de base de ce type d'imagerie repose sur l'application de champs magnétiques non uniformes, sous forme de séquences d'applications successives de gradients de champ magnétique, ceux-ci sont fortement susceptibles de perturber d'autres signaux éventuellement recueillis sur un patient soumis au dispositif de génération d'images, comme les électrocardiogrammes (ECG), Electroencéphalogrammes (EEG)etc. Or, ces signaux sont en principe mesurés en même temps qu'il est procédé à l'enregistrement de vues résultant de la résonance magnétique, notamment pour permettre une surveillance en temps réel du patient pendant les examens IRM, ou pour déclencher des opérations de synchronisation de séquences IRM, ou encore pour les utiliser à l'appui d'autres traitements de signaux en lien ou non avec l'IRM.

Dans ces conditions, des interférences entre les signaux que l'on cherche à collecter et les variations magnétiques dues au dispositif imageur sont inévitables pendant la génération des images, qui brouillent *in fine* le signal recueilli, et en rendent plus problématique la lecture et l'interprétation et la synchronisation de la séquence, quelles que soient les finalités prévues pour le signal mesuré.

Cette question de la perturbation des signaux de surveillance et de contrôle de type ECG lors de la génération d'images par IRM est récurrente du fait de son importance pratique dans le domaine de l'imagerie médicale, notamment mesurée à l'aune de l'importance des fonctions qui peuvent être affectées aux signaux recueillis. Ainsi, par exemple, si le contrôle en continu et en temps réel de l'état du patient est vicié, ou si les opérations de synchronisation et plus généralement les opérations déclenchées par certains états physiologiques mesurés des signaux ne peuvent pas s'effectuer correctement par manque de précision du signal, on comprend aisément les problèmes posés, voire les risques encourus. En particulier, en ce qui concerne les opérations de synchronisation, il peut en résulter une qualité d'image qui n'est pas compatible avec la réalisation d'un diagnostic.

Il est dès lors primordial de maîtriser la forme des signaux recueillis, en temps réel, en supprimant certains artéfacts indésirables ou en séparant ce qui provient de différentes sources en vue d'obtenir au moins un signal clair répondant aux objectifs recherchés. Ainsi, par exemple pour le contrôle du cœur d'un patient soumis à une IRM, il est important de recueillir en temps réel un signal d'ECG avec le moins d'artéfacts possible, et par conséquent de supprimer notamment les artéfacts liés aux gradients de champs magnétiques qui accompagnent les séquences de génération d'images par IRM.

Compte tenu de la prégnance de ce problème, de nombreuses solutions ont déjà été proposées pour débarrasser le signal électrophysiologique, par exemple d'ECG, d'EOG ou d'EEG, de ses artéfacts dus aux variations de champ magnétique, parmi lesquelles le positionnement de l'électronique de traitement à proximité du point de mesure et l'utilisation d'une transmission optique ou radiofréquences insensible aux interférences magnétiques. Il a également été proposé l'utilisation de boucles conductrices proches de l'endroit de mesure pour recueillir des signaux identiques aux interférences, qui sont ensuite soustraites aux signaux mesuré. L'idée de mesurer séparément le signal de l'ECG d'une part, et les interférences d'autre part, par un positionnement adapté des capteurs, a d'ailleurs été reprise, mais cette différence de positionnement représente la limite de la solution, puisque par essence, les interférences mesurées ne sont pas exactement celles qui apparaissent à l'endroit de la mesure du signal de l'ECG et que malgré la différence d'emplacement, le second signal mesuré contient aussi une partie du signal utile.

De nombreuses méthodes de traitement du signal ont par ailleurs été tentées, dont des solutions basées sur le calcul de réponses impulsionnelles aux gradients de champ magnétique, sur la base de la connaissance de leurs courants de génération, pour corriger les artéfacts, et/ou des algorithmes utilisant des méthodes de déconvolution ou des approches adaptatives.

Pour pallier les limitations de ces solutions, notamment dues aux liaisons électroniques entre le module de traitement du signal et l'électronique de commande du dispositif d'IRM, et qui ne tiennent pas ou que très imparfaitement compte de l'existence de courants de Foucault affectant la précision des calculs, le déposant lui-même a développé une solution, décrite par exemple dans le document WO 2008/155488.

Celle-ci consiste, pour l'essentiel, à combiner le signal de l'ECG à la mesure du champ magnétique local tel que fournie par un capteur par exemple à effet Hall. La mesure des variations temporelles du champ magnétique par un ou plusieurs capteurs de ce type est nécessaire pour à la fois simuler puis supprimer les artéfacts générés dans le signal de l'ECG, par ailleurs mesurés par le ou les capteurs. Pour permettre une correction du signal la meilleure possible, un capteur à effet Hall est - selon cette solution - placé le plus proche possible du capteur de l'ECG, dans l'optique de mesurer les variations de champ magnétique qui provoquent en réalité les artéfacts mesurés par ce capteur. La connaissance de ces variations et de leurs conséquences permet un traitement visant à les supprimer en temps réel du signal de l'ECG tel qu'initialement recueilli.

En pratique, cela nécessite de positionner le capteur à effet Hall sur la poitrine du patient, avec pour conséquence nécessaire et inévitable de soumettre ledit capteur à tous les déplacements induits par les mouvements du corps, conscients ou non. Or, les déplacements de la poitrine sont complexes à modéliser, et induisent ou sont décomposables en des rotations du ou des capteurs d'axes X, Y et Z dont il faut en toute rigueur tenir compte pour pouvoir apprécier l'impact du champ magnétique sur les calculs liés aux traitements des signaux.

Dans le contexte particulier à l'invention, avec un dispositif d'imagerie médicale de type IRM, le patient est placé dans un tunnel dont l'axe est colinéaire à la direction du champ magnétique statique produit, typiquement d'une valeur de l'ordre de 1,5 T à 3 T et orienté selon l'axe Z. Les modifications de position du capteur à effet Hall, modifiant du même coup l'orientation relative des directions respectivement du vecteur champ magnétique et de mesure du capteur (d'un angle variable a), ont un impact sur les valeurs mesurées, qui se voient réduites d'un facteur multiplicatif d'une valeur inférieure à 1, typiquement de l'ordre de cos(a).

Cet impact peut être significatif, selon l'amplitude des mouvements de la poitrine, et notamment des déplacements résultant des respirations et des pulsations cardiaques. Bien qu'il soit demandé au patient de rester le plus immobile possible, ces mouvements ne sont pas contrôlables, et ils surviennent dès lors périodiquement au cours des séquences de génération d'images par IRM. Ils modifient en tout état de cause le signal recueilli, et influencent l'évaluation concrète de l'impact des gradients par exemple dans un signal d'ECG, dont on a vu auparavant l'importance. Si les artéfacts liés aux mouvements du patient ne sont pas pris en compte dans le traitement des signaux obtenus, les résultats finaux ne peuvent pas être considérés comme suffisamment fiables, pour la raison développée ci-après.

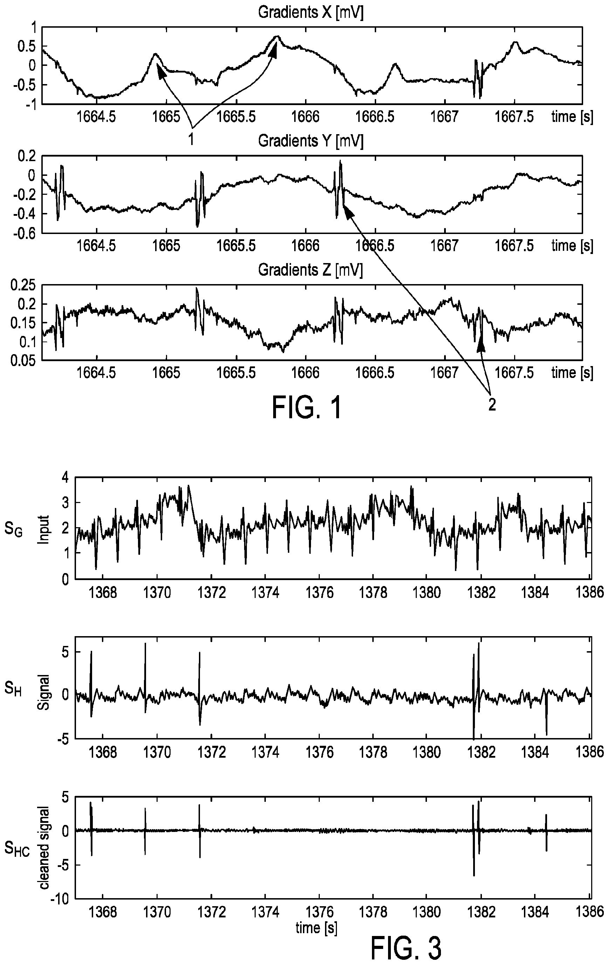

Des traitements de filtrage classiques permettraient en effet en théorie de séparer ces artéfacts des effets des gradients de champs magnétiques, mais les gammes de fréquences des séquences recouvrent en pratique au moins partiellement celles des artéfacts. Cela rend par conséquent l'opération de filtrage assez peu fiable, comme on le verra dans la suite plus en détail en référence à la figure 1. Un tel problème de recouvrement des gammes de fréquences ne se retrouve par exemple pas dans le document US2018/055417, qui traite d'une méthode de mesure de mouvements au moyen d'un dispositif porté par un individu, et comportant un capteur porté par un membre de son corps et destiné à capter les signaux émis par une autre partie du corps. Il est alors possible de déterminer, à l'aide d'une modélisation des distances entre ces composants, une posture ou une attitude du corps. Ce document mentionne l'existence d'un filtrage en temps réel sur les variations des signaux de distance dus aux mouvements, mais dont il faut souligner que le domaine de fréquence est très éloigné des fréquences magnétiques. Le problème de la superposition des fréquences à traiter et des fréquences à filtrer ne s'y pose donc pas.

Or, l'invention vise précisément à remédier à ce problème, et notamment à isoler précisément le gradient de champ magnétique des artéfacts causés par la respiration et les pulsations cardiaques, et plus généralement par tous les déplacements contrôlés ou incontrôlées, conscients ou inconscients, du corps du patient allongé dans le tunnel du dispositif d'IRM. L'objectif est d'obtenir en temps réel une mesure propre, c'est-à-dire débarrassée des artéfacts précédents, du signal du gradient de champ magnétique pendant une séquence d'acquisition d'images par un dispositif IRM. Ledit signal apuré est ensuite classiquement utilisé pour la correction en temps réel du signal par exemple d'ECG, notamment et comme mentionné à des fins de surveillance du patient, ou pour d'autres utilisations de ce signal corrigé et fiable.

Il est à noter que les informations obtenues sur les déplacements imprimés au capteur par les mouvements du patient, telles que séparées des autres signaux via l'invention, peuvent également servir à d'autres usages, comme par exemple la surveillance en temps réel de ce patient à d'autres fins.

A ces effets, l'invention a tout d'abord trait à un procédé de correction en temps réel de mesures de variations du champ magnétique existant par exemple dans un dispositif d'investigation exploitant la résonance magnétique, ledit procédé étant défini dans la revendication indépendante 1.

Deux étapes principales sont donc mises en œuvre dans le cadre du procédé de l'invention, qui impliquent une mesure de déplacements, sinon continue du moins à une fréquence raisonnablement élevée, et un traitement simultané des valeurs mesurées pour opérer un filtrage et permettre d'obtenir un signal résiduel propre en temps réel.

La caractéristique de l'environnement électromagnétique dont les variations sont mesurées est, de manière très directe, le champ magnétique. Cette caractéristique est variable dans l'environnement décrit du fait de la technologie propre à l'IRM, qui nécessite l'application de gradients de champ magnétique pour la génération d'images.

En fait, dans le procédé selon l'invention, la prise de mesures de déplacement ou de vitesse des moyens de mesure de la caractéristique de l'environnement électromagnétique consiste en une succession de mesures d'au moins les rotations ou les vitesses de rotation autour d'au moins un axe de ces moyens de mesure, lesdites mesures faisant l'objet d'un enregistrement de paramètres pour chaque mesure. Une telle mesure notamment des déplacements rotatifs est en effet assez aisée à mettre en œuvre, comme on le verra dans la suite. De fait, les mouvements du corps humain qui sont en cause, typiquement la respiration et les pulsations cardiaques, provoquent des déplacements décomposables en rotations selon plusieurs axes.

De son côté, le filtrage consiste en un calcul en temps réel de la relation entre les paramètres enregistrés pour chaque mesure et un parmi les artéfacts, les paramètres enregistrés conduisant bijectivement à une identification d'un artéfact, chaque artéfact identifié comme correspondants à des paramètres d'une mesure étant ensuite séparé puis supprimé du signal issu de la mesure des variations de la caractéristique de l'environnement électromagnétique. En pratique, une identification puis une séparation desdits artéfacts sont donc effectuées avant suppression. Les artéfacts séparés sont le cas échéant utilisables pour d'autres usages.

Ces étapes de procédé sont réalisées, selon l'invention, par un dispositif de correction en temps réel de mesure des variations du champ magnétique existant par exemple dans un dispositif d'investigation exploitant la résonance magnétique, ledit dispositif selon l'invention étant défini dans la revendication indépendante 3.

Ces système/moyens se combinent pour mettre en œuvre de façon concrète les étapes du procédé de l'invention, et sont respectivement en charge des étapes de mesure et de traitement du signal qui sont l'apanage dudit procédé. A nouveau, la caractéristique de l'environnement électromagnétique dont les variations sont mesurées est le champ magnétique, dans un dispositif d'imagerie médicale à résonnance magnétique fonctionnant avec des séquences d'application de gradients dudit champ magnétique, pour la génération des images du patient.

De fait, selon une possibilité, le système de mesure des déplacements ou des vitesses du corps au voisinage des moyens de mesure de la caractéristique de l'environnement électromagnétique consiste en des moyens de calcul des déplacements ou des vitesses des moyens de mesure de la caractéristique de l'environnement électromagnétique.

Plus précisément encore, ces moyens de calcul peuvent consister en au moins un capteur de position angulaire ou de vitesse angulaire placé au voisinage desdits moyens de mesure de la caractéristique. Selon l'invention, il s'agit d'un capteur de vitesse angulaire consistant en un gyroscope.

De même, selon l'invention, les moyens de mesure de la caractéristique de l'environnement électromagnétique consistent en au moins un capteur à effet Hall.

Enfin, pour ce qui concerne le traitement du signal en vue de la séparation visant à rendre « propre » le signal évalué, les moyens de filtrage adaptatifs sont des moyens de calcul adaptatifs de la relation entre chaque déplacement ou vitesse mesuré(e) et un des artéfacts enregistrés, permettant une identification bijective entre chaque déplacement ou vitesse mesuré(e) et un artéfact, et des moyens de suppression desdits artéfacts du signal issu de la mesure des variations de ladite caractéristique de l'environnement électromagnétique.

Selon l'invention, ces moyens de filtrage peuvent comporter un étage correctif de reconstruction d'un signal corrigé basé sur le signal d'au moins un capteur à effet Hall, à partir d'une part du signal non corrigé et d'autre part d'un signal de correction. Ce signal de correction est composé, pour chaque point d'indice n du signal non corrigé traité, d'un signal bufferisé $U_M(n)$ à partir d'un groupe de M points du signal issu du gyroscope précédant temporellement le point d'indice n traité et d'un signal issu d'un filtre adaptatif alimenté par le signal issu du capteur à effet Hall corrigé et par ledit signal bufferisé.

Selon une configuration possible, la réponse impulsionnelle H(n) du filtre adaptatif comporte une composante calculée en utilisant d'une part le signal issu du capteur à effet Hall corrigé $S_{HC}(n)$, et d'autre part les signaux gyroscopiques bufferisés avec un pas d'avance $\mu$ obéissant à la relation $H(n+1) = H(n) + 2\mu\, S_{HC}(n)U_M(n)$.

L'invention va à présent être décrite plus en détail, en référence aux figures annexées qui illustrent un exemple non limitatif de mise en œuvre de l'invention, et pour lesquelles :

- la figure 1 représente une superposition de diagrammes temporels montrant les signaux mesurés selon les trois axes Y, Y et Z par un capteur à effet Hall placé sur la poitrine d'un patient pendant une séquence d'acquisition d'images par IRM ;
- la figure 2 montre la configuration schématique du circuit de traitement mis en œuvre pour la correction du signal issu d'un capteur à effet Hall ;
- la figure 3 représente un diagramme montrant les signaux émis par un gyroscope, un capteur à effet Hall et, en sortie, le signal corrigé des artéfacts dus aux déplacements du capteur à effet Hall ;
- la figure 4 illustre de manière schématique le traitement final du signal ECG après traitement du signal émanant d'un capteur à effet Hall selon l'invention ;
- la figure 5 montre les diagrammes temporels synchronisés des gradients de champs magnétiques, du signal de l'ECG mesuré et du signal de l'ECG corrigé.

Tout d'abord, pour mesurer l'apport de l'invention, il est important de comprendre que les mouvements du patient changent les mesures : si, à la suite d'un déplacement, un capteur à effet Hall effectue une mesure dans une direction qui fait un angle $\alpha$ avec la direction du champ magnétique principal $B_O$ constant et permanent du dispositif imageur, alors la valeur de champ mesurée $B_{mes}$ sera égale à $B_O \times \cos(a)$ il est par conséquent nécessaire de tenir compte de ces déplacements et de leurs impacts sur les mesures pour aboutir à des traitements des signaux les plus précis possibles.

En référence à la figure 1, les trois diagrammes temporels, pris sur les trois axes X, Y et Z, montrent une acquisition de signaux par capteurs à effet Hall pendant une séquence typique de fonctionnement d'imageur IRM où, pour la production d'images, des gradients de champs magnétiques sont appliqués. Les pulsations cardiaques 1, qui provoquent un léger mouvement de la poitrine, et la respiration, qui occasionne en fait une ondulation de base, produisent des artéfacts qui se mêlent aux gradients de champ magnétique 2 tels que mesurés, même si le patient reste le plus calme et le plus immobile possible. Ces artéfacts affectent évidemment la mesure du signal magnétique variable, et un filtrage classique en vue de séparer leurs effets du signal magnétique ne permet pas d'aboutir à un résultat fiable du fait de la proximité et de la possibilité de recouvrement des gammes de fréquences, qui est manifeste à la vue de la figure 1. Le but de l'invention est donc d'isoler le signal magnétique desdits artéfacts.

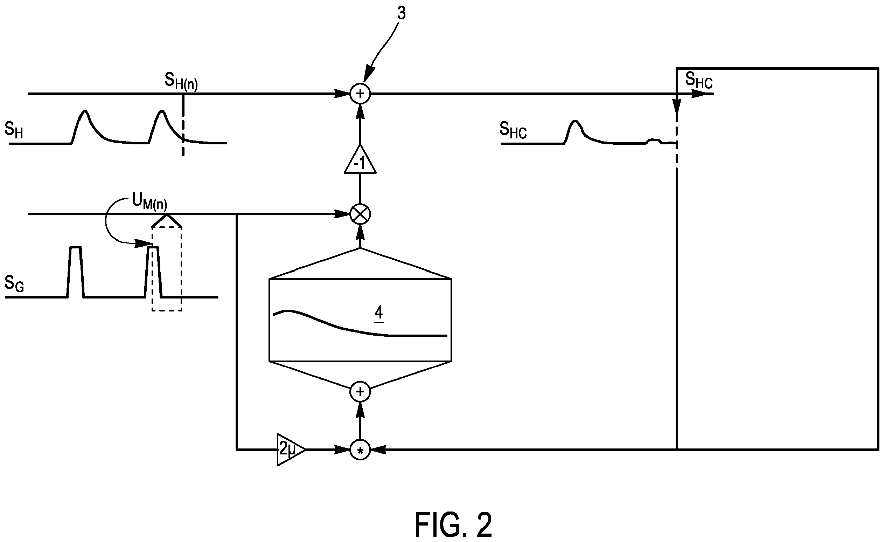

La solution utilisée dans le cadre de l'invention consiste à mesurer les déplacements de chaque capteur à effet Hall, au moyen d'au moins un capteur de mouvement angulaire qui est basé sur un gyroscope en technologie MEMS avec une mesure de vitesse angulaire, placé à cet effet au voisinage immédiat du capteur à effet Hall. Comme montré en figure 2, le signal $S_H$ issu d'un capteur à effet Hall est corrigé en un signal $S_{HC}$ par un étage correctif 3 qui applique en temps réel audit signal $S_H$ un filtrage adaptatif réalisé par un filtre 4. Le filtrage adaptatif s'applique itérativement à un point du signal $S_H(n)$ référencé n et utilise M points notés $U_M(n)$ du signal $S_G$ (les points délimités par le rectangle en traits pointillés, qui délimite l'intervalle temporel sélectionné) issu du gyroscope et bufferisés, M étant défini par l'utilisateur et constituant le nombre de coefficients du filtre 4. Ces M points de la courbe du signal $S_G$ précèdent temporellement, comme cela est visible sur la figure 2, le point $S_H(n)$ de la courbe du signal $S_H$ (voir la ligne verticale en pointillés qui relie le point $S_H(n)$ et la fin de l'intervalle temporel sélectionné). Les coefficients que constituent ces M points sont mis à jour en permanence, et sont utilisés pour ajouter à la réponse impulsionnelle H(n) du filtre 4 une composante calculée en utilisant d'une part le signal issu du capteur à effet Hall corrigé $S_{HC}(n)$, et d'autre part les signaux gyroscopiques bufferisés $U_M(n)$ avec un pas d'avance μ également défini par l'utilisateur, selon la relation :

$$H(n+1) = H(n) + 2\mu\ S_{HC}(n)U_M(n)$$

A chaque étape de mise à jour, la contribution du signal gyroscopique $S_G$ dans le signal $S_H$ issu du capteur à effet Hall est prise en compte, et intégrée dans le filtre 4, puis corrigée au niveau de l'étage correctif 3.

En sortie, le signal $S_{HC}$ est un signal de capteur à effet Hall corrigé de tous les « bruits » parasites indésirés que sont les artéfacts mentionnés auparavant. C'est à présent un signal $S_{HC}$ clair et propre, qui constitue une base correcte pour les traitements et opérations ultérieurs.

La figure 3 montre les différents signaux apparaissant en figure 2, chacun dans un diagramme temporel propre, lesdits diagrammes étant synchronisés, montrant parfaitement le haut niveau et l'efficacité du filtrage effectué. Ainsi, en haut, le signal $S_G$ reflète les déplacements du capteur à effet Hall. Au milieu, le signal $S_H$ correspond à ce que produit le capteur à effet Hall lorsqu'il n'est pas débarrassé de ces artéfacts dus aux mouvements du corps du patient. On y retrouve en effet une base de signaux imputables aux mouvements, de fréquences du même ordre que dans le diagramme du dessus. En bas, suite aux traitements des signaux effectués par le circuit de la figure 2, le signal $S_{HC}$ apparaît débarrassé des artéfacts, de sorte que les gradients de champ impulsés au système pour l'obtention des images par l'IRM sont parfaitement visibles et surtout, faciles à exploiter, d'une manière connue en soi et protégée par ailleurs par le déposant.

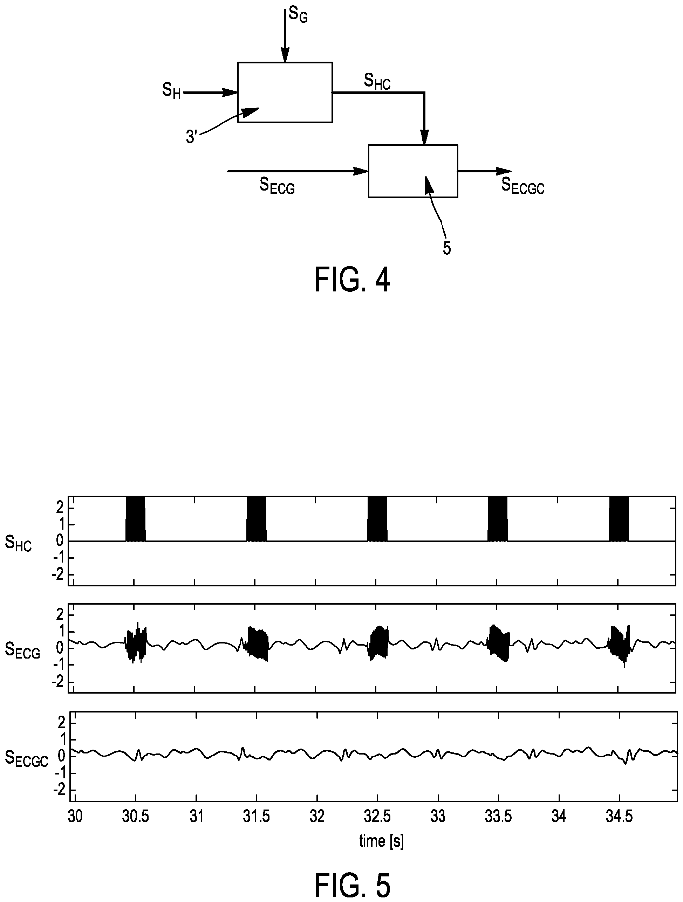

L'intérêt de ces traitements en amont, qui constitue la présente invention proprement dite, est double. Tout d'abord, et comme cela ressort de la figure 4, le signal $S_{HC}$ du capteur à effet Hall tel que corrigé par les étages 3 et 4 (regroupés en un filtre adaptatif global 3' en figure 4) est utilisé pour corriger en temps réel le signal de l'Electrocardiogramme $S_{ECG}$, affecté des artéfacts cette fois dus aux gradients de champ magnétique utilisés pour la génération d'images d'IRM. Cette correction s'effectue dans un autre filtre adaptatif 5 de manière à obtenir in fine un signal $S_{ECGC}$ d'Electrocardiogramme « propre », qui ne constitue qu'un exemple possible de signal électro-physiologique, utilisé en l'espèce pour la démonstration. C'est-à-dire, encore une fois et de manière connue, un signal $S_{ECGC}$ débarrassé des artéfacts liés aux variations de champ magnétique nécessaires à la visualisation des images selon le principe de l'IRM.

De fait, on procède en deux étapes : une fois le signal du capteur à effet Hall débarrassé des bruits liés aux déplacements issus d'événements physiologiques indépendants de la volonté du patient, les seuls signaux qui restent sont ceux qui proviennent des gradients de champ magnétique, qui sont alors utilisables comme signaux de commande pour supprimer les artéfacts de gradient de champ magnétique sur les ECG. C'est ce qui ressort aussi de la figure 5, dont le diagramme supérieur montre la manière dont les gradients affectent la forme d'un signal $S_{HC}$ recueilli en l'occurrence par un capteur à effet Hall et déjà traité - selon l'invention - pour supprimer les effets des mouvements du patient. Le diagramme du milieu montre un signal d'ECG $S_{ECG}$ dont on voit bien, du fait de la synchronisation temporelle des diagrammes, qu'il est affecté par lesdits gradients. Enfin, suite au filtrage dynamique en temps réel réalisé par le filtre adaptatif 5 de la figure 4, le signal $S_{ECGC}$ ne fait plus apparaître que les pulsations cardiaques 6, périodiques et sensiblement débarrassées de tout bruit. La qualité du signal est en tout état de cause telle qu'elle permet une utilisation confortable et fiable dudit signal propre pour surveiller un patient ou pour déclencher, le cas échéant, des opérations additionnelles, via le dispositif à IRM ou autre.

Il est à noter que la possibilité offerte par l'invention d'une séparation claire, dans les signaux obtenus, des différents artéfacts, permet de monitorer le cas échéant la respiration du sujet, ou encore de mesurer les pulsations cardiaques, à des fins plus directement médicales. Parmi les conséquences indirectes de l'invention, on a donc un outil de surveillance plus précis, utilisable durant les séquences d'IRM, et ne nécessitant pas la pose et le contrôle de capteurs supplémentaires.

L'invention ne se limite bien entendu pas aux exemples décrits et expliqués en référence aux figures, mais elle englobe les variantes et versions qui entrent dans la portée des revendications.

## Revendications

1. Procédé de correction en temps réel de mesures de variations du champ magnétique existant par exemple dans un dispositif d'investigation exploitant la résonance magnétique, ledit champ magnétique étant mesuré sur un sujet soumis à investigations par des moyens de mesure solidarisés à une zone du corps susceptible d'être affectée par des déplacements induits par des fonctions naturelles du corps notamment liés à la respiration ou aux battements cardiaques, qui présente pour chaque caractéristique mesurée :

   - une prise de mesures de déplacement et/ou de vitesse des moyens de mesure du champ magnétique ; et
   - un filtrage en temps réel des artéfacts provoqués par lesdits déplacements dans le signal issu de la mesure des variations du champ magnétique **caractérisé en ce que** :

      - le filtrage consiste en un calcul en temps réel de la relation entre les paramètres enregistrés pour chaque mesure et un parmi les artéfacts, les paramètres enregistrés conduisant bijectivement à une identification d'un artéfact, chaque artéfact identifié comme correspondants à des paramètres d'une mesure étant ensuite séparé puis supprimé du signal issu de la mesure des variations du champ magnétique ;
      - le filtrage comporte une correction pour la reconstruction d'un signal corrigé $S_{HC}$ basé sur le signal d'au moins un capteur à effet Hall, à partir d'une part d'un signal non corrigé $S_H$ de mesure des variations du champ magnétique, et d'autre part d'un signal de correction, ledit signal de correction étant composé, pour chaque point d'indice n du signal non corrigé $S_H$ traité, d'un signal bufferisé $U_M(n)$ à partir d'un groupe de M points du signal issu d'au moins un gyroscope $S_G$ précédant temporellement le point $S_H(n)$ et d'un signal issu d'un filtre adaptatif (4) alimenté par le signal corrigé issu du capteur à effet Hall $S_{HC}$ et par ledit signal bufferisé $U_M(n)$.

2. Procédé selon la revendication précédente, **caractérisé en ce que** la prise de mesures de déplacement ou de vitesse des moyens de mesure du champ magnétique consiste en une succession de mesures d'au moins les rotations ou les vitesses de rotation autour d'au moins un axe de ces moyens de mesure, lesdites mesures faisant l'objet d'un enregistrement de paramètres pour chaque mesure.

3. Dispositif de correction en temps réel de mesure des variations du champ magnétique existant par exemple dans un dispositif d'investigation exploitant la résonance magnétique, ledit champ magnétique sur un sujet vivant par des moyens de mesure solidarisés au corps du sujet, dans une zone du corps susceptible d'être affectée par des déplacements induits par des fonctions naturelles du corps notamment liés à la respiration ou aux battements cardiaques comportant au moins un système de mesure des déplacements ou des vitesses du corps au voisinage des moyens de mesure du champ magnétique, et des moyens de filtrage adaptatifs (3') des artéfacts provoqués par lesdits déplacements ou vitesses dans le signal issu de la mesure des variations du champ magnétique, **caractérisé en ce que** :

   - les moyens de filtrage adaptatifs (3') sont des moyens de calcul adaptatifs de la relation entre chaque déplacement ou vitesse mesuré(e) et un des artéfacts enregistrés, permettant une identification bijective entre chaque déplacement ou vitesse mesuré(e) et un artéfact, et des moyens de suppression desdits artéfacts du signal issu de la mesure des variations du champ magnétique ;
   - les moyens de filtrage (3') comportent un étage correctif (3) de reconstruction d'un signal corrigé $S_{HC}$ basé sur le signal d'au moins un capteur à effet Hall, à partir d'une part d'un signal non corrigé $S_H$ et d'autre part d'un signal de correction, ledit signal de correction étant composé, pour chaque point d'indice n du signal non corrigé traité, d'un signal bufferisé $U_M(n)$ à partir d'un groupe de M points du signal issu d'au moins un gyroscope $S_G$ précédant temporellement le point $S_H(n)$ et d'un signal issu d'un filtre adaptatif (4) alimenté par le signal issu du capteur à effet Hall corrigé $S_{HC}$ et par ledit signal bufferisé $U_M(n)$.

4. Dispositif de correction selon la revendication précédente, **caractérisé en ce que** le système de mesure des déplacements du corps au voisinage des moyens de mesure du champ magnétique consiste en des moyens de calcul des déplacements ou des vitesses des moyens de mesure du champ magnétique.

5. Dispositif de correction selon la revendication précédente, **caractérisé en ce que** les moyens de calcul des déplacements des moyens de mesure champ magnétique consistent en au moins un capteur de position angulaire ou de vitesse angulaire placé au voisinage desdits moyens de mesure de la caractéristique.

6. Dispositif de correction selon l'une des revendications 3 à 5, **caractérisé en ce que** la réponse impulsionnelle H(n) du filtre (4) comporte une composante calculée en utilisant d'une part le signal issu du capteur à effet Hall corrigé $S_{HC}(n)$, et d'autre part les signaux gyroscopiques bufferisés $U_M(n)$ avec un pas d'avance $\mu$ obéissant à la relation $H(n+1) = H(n) + 2\mu\, S_{HC}(n)U_M(n)$.

**Patentansprüche**

1. Verfahren zur Korrektur in Echtzeit von Messungen von Variationen des vorhandenen Magnetfelds beispielsweise in einer Untersuchungsvorrichtung, die die Magnetresonanz nutzt, wobei das Magnetfeld an einem Subjekt gemessen wird, das Untersuchungen durch Messmittel unterzogen wird, die mit einer Zone des Körpers fest verbunden sind, die von Verschiebungen betroffen sein könnte, die von natürlichen Funktionen des Körpers hervorgerufen werden, die insbesondere mit der Atmung oder mit dem Herzschlag verbunden sind, das für jedes gemessene Merkmal aufweist:

- eine Durchführung von Verschiebungs- und/oder Geschwindigkeitsmessungen der Messmittel des Magnetfelds; und
- eine Filterung in Echtzeit der von den Verschiebungen im Signal, das von der Messung der Variationen des Magnetfelds ausgegeben wird, hervorgerufenen Artefakte;

**dadurch gekennzeichnet, dass**:

- die Filterung aus einer Berechnung in Echtzeit des Verhältnisses zwischen den für jede Messung gespeicherten Parametern und von einem der Artefakte besteht, wobei die gespeicherten Parameter bijektiv zu einer Identifizierung eines Artefakts führen, wobei jedes Artefakt, das als Parametern einer Messung entsprechend identifiziert wird, danach vom Signal, das von der Messung der Variationen des Magnetfelds ausgegeben wird, getrennt und dann gelöscht wird;
- die Filterung eine Korrektur für die Rekonstruktion eines korrigierten Signals $S_{HC}$ aufweist, basierend auf dem Signal von mindestens einem Sensor mit Hall-Effekt, ausgehend, zum einen, von einem nicht korrigierten Messsignal $S_H$ der Variationen des Magnetfelds und, zum anderen, einem Korrektursignal, wobei das Korrektursignal für jeden Indexpunkt n des verarbeiteten nicht korrigierten Signals $S_H$ aus einem gepufferten Signal $U_M(n)$ ausgehend von einer Gruppe von M Punkten des Signals, das von mindestens einem Gyroskop $S_G$ ausgegeben wird, das zeitlich vor dem Punkt $S_H(n)$ liegt, und aus einem Signal zusammengesetzt ist, das von einem adaptiven Filter (4) ausgegeben wird, der von dem korrigierten Signal $S_{HC}$, das von dem Sensor mit Hall-Effekt ausgegeben wird, und von dem gepufferten Signal $U_M(n)$ versorgt wird.

2. Verfahren nach vorangehendem Anspruch, **dadurch gekennzeichnet, dass** die Durchführung von Verschiebungs- oder Geschwindigkeitsmessungen der Messmittel des Magnetfelds aus einer Abfolge von Messungen von mindestens den Rotationen oder den Rotationsgeschwindigkeiten um mindestens eine Achse dieser Messmittel besteht, wobei die Messungen Gegenstand einer Speicherung von Parametern für jede Messung sind.

3. Vorrichtung zur Korrektur in Echtzeit von Messung der Variationen des vorhandenen Magnetfelds beispielsweise in einer Untersuchungsvorrichtung, die die Magnetresonanz nutzt, wobei das Magnetfeld an einem lebenden Subjekt von Messmitteln gemessen wird, die mit dem Körper des Subjekts fest verbunden sind, in einer Zone des Körpers, die von Verschiebungen betroffen sein könnte, die von natürlichen Funktionen des Körpers hervorgerufen werden, die insbesondere mit der Atmung oder mit dem Herzschlag verbunden sind, aufweisend mindestens ein System zum Messen der Verschiebungen oder der Geschwindigkeiten des Körpers in der Nähe der Messmittel des Magnetfelds, und adaptive Filtermittel (3') der Artefakte, die von den Verschiebungen oder Geschwindigkeiten in dem Signal hervorgerufen werden, das von der Messung der Variationen des Magnetfelds ausgegeben wird, **dadurch gekennzeichnet, dass**:

- die adaptiven Filtermittel (3') adaptive Mittel zur Berechnung des Verhältnisses zwischen jeder gemessenen Verschiebung oder Geschwindigkeit und einem der gespeicherten Artefakte sind, wodurch eine bijektive Identifizierung zwischen jeder gemessenen Verschiebung oder Geschwindigkeit und einem Artefakt ermöglicht wird, und Löschmittel der Artefakte des von der Messung der Variationen des Magnetfelds ausgegebenen Signals;

- die Filtermittel (3') eine Korrekturstufe (3) zur Rekonstruktion eines korrigierten Signals $S_{HC}$ aufweisen, das auf dem Signal von mindestens einem Sensor mit Hall-Effekt basiert, ausgehend, zum einen, von einem nicht korrigierten Signal $S_H$ und, zum anderen, einem Korrektursignal, wobei das Korrektursignal für jeden Indexpunkt n des verarbeiteten nicht korrigierten Signals aus einem gepufferten Signal $U_M(n)$ ausgehend von einer Gruppe von M Punkten des Signals, das von mindestens einem Gyroskop $S_G$ ausgegeben wird, das zeitlich vor dem Punkt $S_H(n)$ liegt, und aus einem Signal zusammengesetzt ist, das von einem adaptiven Filter (4) ausgegeben wird, der von dem korrigierten Signal, das von dem Sensor mit Hall-Effekt $S_{HC}$ ausgegeben wird, und von dem gepufferten Signal $U_M(n)$ versorgt wird.

4. Korrekturvorrichtung nach vorangehendem Anspruch, **dadurch gekennzeichnet, dass** das Messsystem der Verschiebungen des Körpers in der Nähe der Messmittel des Magnetfelds aus Mitteln zur Berechnung der Verschiebungen oder der Geschwindigkeiten der Messmittel des Magnetfelds besteht.

5. Korrekturvorrichtung nach vorangehendem Anspruch, **dadurch gekennzeichnet, dass** die Mittel zur Berechnung der Verschiebungen der Messmittel des Magnetfelds aus mindestens einem Sensor der Winkelposition oder der Winkelgeschwindigkeit bestehen, der in der Nähe der Messmittel des Merkmals platziert ist.

6. Korrekturvorrichtung nach einem der Ansprüche 3 bis 5, **dadurch gekennzeichnet, dass** die Impulsantwort H(n) des Filters 4 eine berechnete Komponente durch Nutzung zum einen des korrigierten Signals $S_{HC}(n)$ aufweist, das von dem Sensor mit Hall-Effekt ausgegeben wird, und zum anderen der gepufferten Gyroskopsignale $U_M(n)$, mit einem Schritt $\mu$ voraus, der dem Verhältnis H(n+1) = H(n) + 2$\mu$ $S_{HC}(n)U_M(n)$ entspricht.

**Claims**

1. A method for real-time correction of measurements of variations of the magnetic field existing for example in an investigation device using magnetic resonance, said magnetic field being measured on a subject who is subject to investigations by measurement means secured to an area of the body able to be affected by movements caused by natural functions of the body, in particular related to breathing or heartbeat, which includes, for each measured characteristic:

- taking measurements of movement and/or speed from the magnetic field measuring means; and
- real-time filtering of the artifacts caused by said movements in the signal resulting from the measurements of the magnetic field variations;

**characterized in that**:

- the filtering consists in a real-time calculation of the relationship between the parameters recorded for each measurement and one among the artifacts, the recorded parameters bijectively leading to an identification of an artifact, each artifact identified as corresponding to parameters of a measurement next being separated, then eliminated from the signal resulting from the measurement of the magnetic field variations;
- the filtering includes a correction for the reconstruction of a corrected signal $S_{HC}$ based on the signal from at least one Hall effect sensor, from on the one hand an uncorrected signal $S_H$ for measuring variations in the magnetic field, and on the other hand a correction signal, said correction signal being made up, for each point with index n of the treated uncorrected signal $S_H$, of a buffered signal $U_M(n)$ from a group of M points of the signal resulting from at least one gyroscope $S_G$ temporally preceding the point $S_H(n)$ and a signal resulting from an adaptive filter (4) applied by the corrected signal resulting from the Hall effect sensor $S_{HC}$ and by said buffered signal $U_M(n)$.

2. The method according to the preceding claim, **characterized in that** the taking of measurements of movement and/or speed from the magnetic field measuring means consists of a series of measurements of at least the rotations or the speeds of rotation about at least one axis of these measuring means, said measurements being subject to a recording of parameters for each measurement.

3. A device for real-time correction of the measurement of variations in the magnetic field existing for example in an investigation device using magnetic resonance, said magnetic field on a living subject by measuring means secured to the body of the subject, in an area of the body able to be affected by movements caused by natural functions of the body in particular related to breathing or to heartbeats including at least one system for measuring movements or speeds of the body near the means for measuring the magnetic field, and adaptive filtering means (3') for filtering artifacts caused by said movements or speeds in the signal resulting from the measurement of the variations of the magnetic field, **characterized in that**:

- the adaptive filtering means (3') are adaptive calculating means for calculating the relationship between each measured movement or speed and one of the recorded artifacts, allowing a bijective identification between each measured movement or speed and an artifact, and means for eliminating said artifacts from the signal resulting from the measurement of the magnetic field variations;

- the filtering means (3') include a corrective stage (3) for reconstruction of a corrected signal $S_{HC}$ based on the signal from at least one Hall effect sensor, from, on the one hand, an uncorrected signal $S_H$ and, on the other hand, a correction signal, said correction signal being made up, for each point with index n of the processed uncorrected signal, of a buffered signal $U_M(n)$ from a group of M points of the signal resulting from at least one gyroscope $S_G$ temporally preceding the point $S_H(n)$ and of a signal resulting from an adaptive filter (4) applied by the signal resulting from the corrected Hall effect sensor $SH_C$ and by said buffered signal $U_M(n)$.

4. The correction device according to the preceding claim, **characterized in that** the system for measuring movements of the body near the magnetic field measuring means consists of means for calculating movements or speeds of the magnetic field measuring means.

5. The correction device according to the preceding claim, **characterized in that** the means for calculating movements of the magnetic field measuring means consists of at least one angular position or angular speed sensor placed near said means for measuring the characteristic.

6. The correction device according to one of claims 3 to 5, **characterized in that** the impulse response H(n) of the filter (4) includes a component calculated by using, on the one hand, the signal resulting from the corrected Hall effect sensor $S_{HC}(n)$, and on the other hand, the buffered gyroscopic signals $U_M(n)$ with an advance pitch $\mu$ obeying the relationship $H(n+1) = H(n) + 2\mu\, S_{HC}(n)U_M(n)$.

FIG. 1

FIG. 3

FIG. 2

FIG. 4

FIG. 5

**EP 3 552 545 B1**

## RÉFÉRENCES CITÉES DANS LA DESCRIPTION

**Documents brevets cités dans la description**

- WO 2008155488 A
- US 2018055417 A